# EUROPEAN PATENT APPLICATION

(11) **EP 4 792 819 A1**
(43) Date of publication of application: **19.08.2026**
(21) Application number: 25158356.3
(22) Date of filing: 17.02.2025
(51) Int. Cl.: A61K 33/06, A61K 33/30, A61K 33/34, A61K 33/38, A61P 1/00

(54) **NITROGEN OXIDE COMPOUND FOR USE IN A METHOD OF TREATING AND/OR PREVENTING DYSBACTERIOSIS**

(71) Applicant: Boves, Axel, 47906 Kempen (DE)
(72) Inventor: Boves, Axel, 47906 Kempen (DE)
(74) Representative: Maiwald GmbH

(57) **Abstract**

The present disclosure relates to a nitrate compound for use in a method of treating and/or preventing a dysbacteriosis in a subject, in particular in an animal subject, such as a pig or piglet. A nitrogen oxide compound as disclosed herein may be used as a dietary supplement, e.g. as an additive in animal feed or in drink, has a positive influence on the performance. This includes in improved feed, improved feed conversion, and reduced load with pathogenic bacteria.

## Description

### TECHNICAL FIELD

The present disclosure relates to a nitrogen oxide compound for use in a method of treating and/or preventing a dysbacteriosis in a subject, in particular in an animal subject, such as a pig or piglet.

### BACKGROUND OF THE INVENTION

Dysbacteriosis (also referred to as dysbiosis) is a condition characterized by an imbalance in the microbial community within the gastrointestinal (GI) tract of the subject, such as a human or animal subjects. In that, the microbiome is disrupted leading to changes in the functional composition, metabolic activity, or local distribution.

Dysbacteriosis can lead to impaired gut health, affecting digestion, nutrient absorption, immune response, and overall health and performance.

The disease and disease progression typically involves an overgrowth of harmful bacteria, a reduction in beneficial microbes, or both, leading to disturbances in the gut microbiome's balance.

However, a healthy gut microbiome is of utmost importance for digesting nutrients, such as fibers and starches, producing short-chain fatty acids that maintain gut health, and for preventing the growth of pathogenic bacteria. In dysbacteriosis, there is a decrease in beneficial bacteria (e.g., *Lactobacillus* and *Bifidobacterium*), while pathogenic bacteria overgrow thereby producing toxins and inflammatory compounds. This process not only triggers intestinal inflammation but also damages the gut epithelium, and increases gut permeability.

In that, dysbacteriosis usually manifests in the following symptoms:

Frequently, diarrhea (often persistent and watery), or loose stools, or inconsistent feces are observed. Also, dysbacteriosis may reduce the growth of the subject, e.g. the feed efficiency of fattening animals is reduced. Furthermore, and increased susceptibility to infections (including bacterial infections) due to the weakened gut function is observed. In extreme cases, dysbacteriosis may lead to a so-called "entero-haemorrhagic syndrome" (EHS). EHS occurs when the intestine of a subject, such as a pig, fills with gas causing the gas filled intestinal loops to rise and twist. This interrupts the blood supply; the blood is trapped in the intestine. As a result, the intestinal tissue dies quickly and becomes dark in color. The dying intestinal wall allows bacteria and toxins to migrate out of the intestine, leading to shock and circulatory failure of the subject, to die suddenly.

Often, dysbacteriosis also results in behavioral changes, such as lethargy, decreased feed intake, increased restlessness, aggression, and the like. For example, in fattening pigs, an increased aggression and cannibalism (in particular biting each other's tails) may be observed.

As a consequence, subjects suffering from dysbacteriosis often suffer from bacterial infections, such as infections with bacteria (including *salmonella, Lawsonia intracellularis, Clostridium perfringens, Clostridium botulinum, Brachyspira hyodysenteriae,* and/or *Escherichia coli*), growth retardation due to malabsorption of nutrients, chronic inflammation leading to systemic effects, such as metabolic stress, increased mortality, and SINS.

SINS (swine inflammation and necrosis syndrome) is a further consequence of dysbacteriosis and refers to a syndrome resulting from the combined presence and signs of clinical inflammation and dead tissue in the acral areas. SINS particularly affects the tail base, tail tip, ears, coronary bands, heels, soles, claw walls, teats, navel, and face and can be observed in suckling piglets, weaners, and finishing pigs. Clinically, SINS appears in different body parts, such as loss of bristles, swellings, reddening, rhagades, exudations, bleeding, necrosis, and ring-shaped constrictions. In that, the tail and the tail base are usually particularly affected by said symptoms.

In particular, the following risk factors have been identified:

Often, abrupt changes in diet (e.g., switching from milk to solid feed of fattening animals, such as piglets upon weaning) and/or weaning stress in piglets may cause dysbacteriosis, in particular severe diarrhea. Also, prolonged or excessive use of antibiotics can disrupt the microbiota by killing beneficial bacteria allowing pathogenic bacteria, such as *Brachyspira hyodysenteriae, Escherichia coli, Clostridium perfringens,* and *Salmonella* to outcompete beneficial bacteria. Furthermore, poor hygiene in animal housing facilities poor feed hygiene may contribute to the risk of dysbacteriosis.

Therefore, it is frequently recommended to avoid abrupt changes in diets, and/or to grind the grains of an animal feed as coarse as possible as this is thought to have overall positive effects on the digestive system. In this way, more undigested feed reaches the large intestine and is fermented there, thus increasing the formation of medium-chain fatty acids which may prevent or inhibit the proliferation of pathogenic bacteria, such as Clostridia. Furthermore, it is recommended to supplement probiotics (e.g., *Lactobacillus* and *Bifidobacterium* strains) to restore beneficial microbes by promoting the growth of beneficial bacteria. Alternatively or additionally, it is recommended to supplement animal feed with short- and medium-chain fatty acids directly.

Also, the use of antibiotics, improving housing conditions and hygiene of fattening animals, such as pigs, and/or post-weaning care using feed additives like zinc oxide is recommended for managing or preventing dysbacteriosis.

In conclusion, dysbacteriosis in fattening animals (e.g., pigs) and other subjects is a multifactorial condition that can lead to significant economic losses due to poor growth performance, increased morbidity, and higher mortality rates. Also, infections being a consequence of dysbacteriosis may require increased veterinary efforts, vaccinations, and use of antibiotics. Thus, early diagnosis, appropriate management, and maintaining gut microbial balance are key to managing dysbacteriosis and ensuring optimal animal well-being and performance.

### SUMMARY OF THE INVENTION

However, the above recommendations are not always effective. Therefore, there is a strong need for methods of efficiently managing, treating and/or preventing dysbacteriosis in fattening animals to improve well-being of animals and meat quality, and reduce the use of antibiotics and other medicines, and optimize the carbon footprint of meat industry.

The inventor of the present disclosure has surprisingly found that nitrogen oxide compounds, in particular nitrate ions and nitrate compounds and/or nitrite ions and nitrite compounds, are useful in the treatment and/or prevention of dysbacteriosis and its symptoms.

Thus, in one aspect, the present disclosure is directed to a nitrogen oxide compound for use in a method of treating and/or preventing a dysbacteriosis in a subject.

### DETAILED DESCRIPTION OF THE INVENTION

In one aspect, the present disclosure is directed to a nitrate compound for use in a method of treating and/or preventing a dysbacteriosis in a subject.

In that, the inventor of the present disclosure surprisingly realized that nitrogen oxide compounds, in particular nitrates and nitrate compounds and/or nitrites and nitrite compounds, have positive properties in the management, i.e. treatment and/or prevention of dysbacteriosis in fattening animals and/or breeding animals, in particular pigs.

The inventor of the present disclosure surprisingly found that a nitrogen oxide compound as disclosed herein used as a dietary supplement, e.g. as an additive in animal feed or in drink, has a positive influence on the performance. This includes in improved feed performance (such as an increased lean meat value and low fat content of fattened animals), improved feed conversion, and reduced load with pathogenic bacteria including but not limited to salmonella, *Lawsonia intracellularis* (a pathogen that is commonly observed in herds of fattening animals causing proliferative inflammation of the ileum mucosa (ileitis) and/or proliferative hemorrhagic enteropathy), *Escherichia coli* (which may be harmful and cause diarrhea and increased mortality, in particular in young piglet), *Brachyspira hyodysenteriae* (which may cause swine dysentery), and *Clostridiae* (which may cause enterotoxemia and necrotic enteritis, neonathal diarrhea, and the like).

The inventor of the present disclosure has surprisingly found that dysbacteriosis can be efficiently treated and/or prevented by administering a nitrogen oxide compound as disclosed herein.

In that, it was surprisingly observed that administering a nitrogen oxide compound to a subject results in a balanced gut microbiome which strengthens the gut lining, reduces the risk of "leaky gut", which can allow harmful pathogens and toxins to enter the bloodstream of a subject. Instead, the growth of beneficial bacteria in the gut is not altered or even stimulated, helping the subjects fight infections and inflammation more effectively (see example 1).

Independently, it was found that administering the nitrogen oxide compound as disclosed herein to a subject facilitates the digestion and absorption of nutrients, supporting optimal growth and feed conversion or feed efficiency. In other words, it was surprisingly found that subjects, such as piglets, can grow steadily without the setbacks caused by malnutrition or infections linked to an imbalanced gut, as is shown in examples 1 to 3.

As the feed is costly and feed production is associated with the emission of large quantities of carbon dioxide, the present disclosure also enables significant cost savings due to an increased feed efficiency and reduces the carbon footprint of meat production.

The inventor of the present disclosure also found that dysbacteriosis-related illnesses, such as diarrhea and SINS, can be efficiently prevented and/or treated thus reducing the number of piglet deaths, use of antibiotics, and veterinary costs.

Furthermore, dysbacteriosis often requires intervention with antibiotics, but frequent use can lead to antibiotic resistance. Controlling gut health and treating and/or preventing dysbacteriosis as disclosed herein reduces the need for such interventions, aligning with global efforts to curb antimicrobial resistance.

Independently, it has been surprisingly found that also the meat quality is improved (see example 1).

### The nitrogen oxide compound

According to a preferred embodiment of the present disclosure, the nitrogen oxide compound (for use in a method of treating and/or preventing a dysbacteriosis in a subject) is a nitrate compound or a nitrite compound. According to a preferred embodiment of the present disclosure, the nitrogen oxide compound (for use in a method of treating and/or preventing a dysbacteriosis in a subject) is a nitrate compound. According to a preferred embodiment of the present disclosure, the nitrogen oxide compound (for use in a method of treating and/or preventing a dysbacteriosis in a subject) is a nitrite compound.

### The nitrate compound

According to a particularly preferred embodiment of the present disclosure, the nitrogen oxide compound is a nitrate compound.

According to a further preferred embodiment of the present disclosure, the nitrate compound is a nitrate salt or a free nitrate ion.

According to a preferred embodiment of the present disclosure, the nitrate compound is a nitrate salt or a free nitrate ion. In other words, the nitrate compound may comprise or consist of one or more nitrate ions (NO₃⁻) and a non-toxic cation, preferably a pharmaceutically acceptable cation.

It is understood that nitrate is an anion (NO₃⁻). As such, nitrate is essential for plant growth but is generally considered a concern when found in excessive level in soil, water, and food. Sources of nitrates may be synthetic fertilizers, livestock manure, especially from large-scale farming, is produced by certain bacteria or release when plants decompose, eventually forming nitrates. Thus, it is understood that the nitrate compound may be a salt of nitrate.

According to another preferred embodiment of the present disclosure, the nitrate compound is a metal nitrate. According to another preferred embodiment of the present disclosure, the nitrate compound is an alkali nitrate, or earth alkali nitrate. In that, it is understood that alkali nitrates and earth alkali nitrates are readily available from commercial suppliers, and alkali or earth alkali cations are mostly considered safe in foods.

According to a further preferred embodiment of the present disclosure, the nitrate compound is selected from the group consisting of ammonium nitrate (NH₄NO₃), potassium nitrate (KNO₃), sodium nitrate (NaNO₃), calcium nitrate (Ca(NO₃)₂), magnesium nitrate (Mg(NO₃)₂), zinc nitrate (Zn(NO₃)₂), copper nitrate (Cu(NO₃)₂), silver nitrate (AgNO₃), and a mixture thereof.

According to another preferred embodiment of the present disclosure, the nitrate compound is a free nitrate ion. It is understood that the nitrate compound may be administered to a subject in an aqueous solution. In such case, it may not have a cation directly associated with it. In such case, the nitrate compound may be referred to as a free nitrate ion.

It is understood that each of potassium, sodium, calcium, and magnesium cations are essential ions in diet and safe in food. In that, sodium (Na⁺) is crucial for maintaining fluid balance, nerve transmission, and muscle function; potassium (K⁺) is critical for maintaining normal blood pressure, nerve function, and muscle contraction; calcium (Ca²⁺) is critical for bone health, nerve function, and blood clotting; magnesium (Mg²⁺) supports muscle and nerve function, energy production, and blood pressure regulation. In other words, the aforementioned nitrate salts may be both safe and exhibit further health benefits.

According to a yet further preferred embodiment of the present disclosure, the nitrate compound is potassium nitrate (KNO₃), or sodium nitrate (NaNO₃), or a mixture thereof. In that, it is noted that both potassium nitrate (KNO₃) and sodium nitrate (NaNO₃) are both commercially readily available and costs of the management of dysbacteriosis (i.e., treatment and/or prevention thereof) are reduced.

According to another preferred embodiment, the nitrate compound is ammonium nitrate.

The inventor of the present disclosure has surprisingly found that dysbacteriosis can be efficiently treated and/or prevented by administering a nitrate compound as disclosed herein and as shown in examples 1 to 3.

### The nitrite compound

According to a preferred embodiment of the present disclosure, the nitrogen oxide compound is nitrite compound.

According to another preferred embodiment of the present disclosure, the nitrite compound is a nitrite salt or a free nitrite ion. In other words, the nitrite compound may comprise or consist of one or more nitrite ions (NO₂⁻) and a non-toxic cation, preferably a pharmaceutically acceptable cation.

It is understood that nitrite is an anion (NO₂⁻). As such, nitrite is generally considered a concern when found in excessive level in soil, water, and food. Sources of nitrites may be agricultural runoffs, synthetic fertilizers, or wastewater of industrial and chemical sources. Thus, it is understood that the nitrite compound may be a salt of nitrite.

According to another preferred embodiment of the present disclosure, the nitrite compound is a metal nitrite. According to another preferred embodiment of the present disclosure, the nitrite compound is an alkali nitrite, or earth alkali nitrite. In that, it is understood that alkali nitrites and earth alkali nitrites are readily available from commercial suppliers, and alkali or earth alkali cations are mostly considered safe in foods.

According to a further preferred embodiment of the present disclosure, the nitrite compound is selected from the group consisting of ammonium nitrite (NH₄NO₂) potassium nitrite (KNO₂), sodium nitrite (NaNO₂), calcium nitrite (Ca(NO₂)₂), magnesium nitrite (Mg(NO₂)₂), zinc nitrite (Zn(NO₂)₂), copper nitrite (Cu(NO₂)₂), silver nitrite (AgNO₂), and a mixture thereof.

According to another preferred embodiment of the present disclosure, the nitrite compound is a free nitrite ion. It is understood that the nitrite compound may be administered to a subject in an aqueous solution. In such case, it may not have a cation directly associated with it. In such case, the nitrite compound may be referred to as a free nitrite ion.

According to a yet further preferred embodiment of the present disclosure, the nitrite compound is potassium nitrite (KNO₂), or sodium nitrite (NaNO₂), or a mixture thereof. In that, it is noted that both potassium nitrite (KNO₂) and sodium nitrite (NaNO₂) are both commercially readily available and costs of the management of dysbacteriosis (i.e., treatment and/or prevention thereof) are reduced.

### The subject to be treated

According to another preferred embodiment of the present disclosure, the subject is an animal.

According to another preferred embodiment of the present disclosure, the subject is a fattening animal. According to another preferred embodiment of the present disclosure, the subject is a breeding animal, preferably a breeding sow. According to another preferred embodiment of the present disclosure, the subject is a pet, such as a dog or a cat.

According to a further preferred embodiment of the present disclosure, the subject is not a ruminant. In this, it is noted that the reduction of nitrate to nitrite can be higher in ruminants than in non-ruminants which could potentially reduce the therapeutic effect of the nitrogen oxide compound.

According to a yet further preferred embodiment of the present disclosure, the subject is a pig. The age and/or body weight of the pig are not particularly limited.

According to another preferred embodiment of the present disclosure, the subject is a piglet, such as a piglet at weaning. Typically, a piglet weighs about 7 kg, such as 4.5 kg to 9 kg at weaning.

According to another preferred embodiment of the present disclosure, the subject is a pig in the nursery stage. In the nursery stage, a pig increases its weight from about 7 kg to about 30 kg. In other words, the pig in the nursery stage weighs about 20 kg to about 30 kg. A pig in the nursery stage drinks about 2.5 L of water per day.

According to another preferred embodiment of the present disclosure, the subject is a pig in the grower stage (also referred to as early growing period). In the grower stage, a pig increases its weight from about 30 kg to about 70 kg. In other words, the pig in the grower stage weighs about 60 kg to about 70 kg. A pig in the grower stage drinks about 7 L of water per day.

According to another preferred embodiment of the present disclosure, the subject is a pig in the finisher stage (also referred to as the final growing period). In the finisher stage, a pig increases its weight from about 70 kg to about 100 kg. In other words, the pig in the grower stage weighs about 90 kg to about 110 kg. A pig in the finisher stage drinks about 10 L of water per day.

According to another preferred embodiment of the present disclosure, the subject is a breeding sow. A breeding sow has a typical weight in the range of from about 150 kg to about 250 kg. A breeding sow drinks about 15 L to about 25 L of water per day

In other words, it is understood that a pig may drink about 1 L of water per day per 10 kg of body weight.

The nitrogen oxide compound, such as the nitrate compound as disclosed herein and/or the nitrite compound as disclosed herein, according to the present disclosure may be administered to a subject at any one of the above stages.

According to another preferred embodiment of the present disclosure, the subject is a horse.

According to another preferred embodiment of the present disclosure, the subject is a poultry. According to a further preferred embodiment of the present disclosure, the subject is a chicken, or a turkey, or a duck, or a goose, or a quail, or a pigeon, or a falcon.

### Administration of nitrogen oxide compound

According to another preferred embodiment of the present disclosure, the nitrogen oxide compound is administered orally.

The inventor of the present disclosure found that the nitrogen oxide compound may be conveniently administered orally thus reducing the need and costs for a veterinary professional.

According to another preferred embodiment of the present disclosure, the nitrogen oxide compound is administered with a meal and/or with drink.

According to another preferred embodiment of the present disclosure, the nitrogen oxide compound is administered with a meal. In that, it is possible to mix the nitrogen oxide compound with food. For example, the nitrogen oxide compound may be mixed with animal feed. During feeding, the nitrogen oxide compound is ingested with the food and thus reaches the gastrointestinal tract.

According to another preferred embodiment of the present disclosure, the nitrogen oxide compound is mixed with animal feed. According to another preferred embodiment of the present disclosure, the nitrite compound is mixed with animal feed. According to a further preferred embodiment of the present disclosure, the nitrate compound is mixed with animal feed.

According to a further preferred embodiment of the present disclosure, the nitrogen oxide compound is provided as a composition comprising animal feed and the nitrogen oxide compound. According to another preferred embodiment of the present disclosure, the nitrite compound is provided as a composition comprising animal feed and the nitrite compound. According to a further preferred embodiment of the present disclosure, the nitrate compound is provided as a composition comprising animal feed and the nitrate compound.

According to a further preferred embodiment of the present disclosure, the nitrate compound is provided as a composition comprising animal feed and the nitrate compound, wherein the composition comprises the nitrate compound in an amount in the range of from about 0.5 g/kg to about 1.5 g/kg based on the total weight of the composition. According to a further preferred embodiment of the present disclosure, the nitrate compound is provided as a composition comprising animal feed and the nitrate compound, wherein the composition comprises the nitrate compound in an amount in the range of from about 0.8 g/kg to about 1.1 g/kg based on the total weight of the composition. According to a preferred embodiment of the present disclosure, the composition is a dry animal feed (about 88 % dry mass).

According to a further preferred embodiment of the present disclosure, the nitrate compound is provided as a composition comprising animal feed and the nitrate compound, wherein the composition comprises the nitrate compound in an amount of about 0.5 g/kg or more based on the total weight of the composition. According to a further preferred embodiment of the present disclosure, the nitrate compound is provided as a composition comprising animal feed and the nitrate compound, wherein the composition comprises the nitrate compound in an amount of about 0.8 g/kg or more based on the total weight of the composition.

According to another preferred embodiment of the present disclosure, the nitrogen oxide compound is administered with a drink. In that, it is possible to dissolve the nitrogen oxide compound in a drink, such as water. For example, the nitrogen oxide compound may be dissolved in water. During drinking, the nitrogen oxide compound is ingested and thus reaches the gastrointestinal tract.

According to another preferred embodiment of the present disclosure, the nitrite compound is administered with a drink. In that, it is possible to dissolve the nitrite compound in a drink, such as water. For example, the nitrite compound may be dissolved in water. During drinking, the nitrite compound is ingested and thus reaches the gastrointestinal tract.

According to a further preferred embodiment of the present disclosure, the nitrate compound is administered with a drink. In that, it is possible to dissolve the nitrate compound in a drink, such as water. For example, the nitrate compound may be dissolved in water. During drinking, the nitrate compound is ingested and thus reaches the gastrointestinal tract.
According to another preferred embodiment of the present disclosure, the nitrate compound is mixed with animal feed. According to a further preferred embodiment of the present disclosure, the nitrate compound is provided as a composition comprising animal feed and the nitrate compound. According to a further preferred embodiment of the present disclosure, the nitrate compound is provided as an aqueous solution comprising the nitrate compound, wherein the aqueous solution comprises the nitrate compound in an amount in the range of at least 100 mg/L. According to a further preferred embodiment of the present disclosure, the nitrate compound is provided as an aqueous solution comprising the nitrate compound in a concentration of at least 400 mg/L. According to a further preferred embodiment of the present disclosure, the nitrate compound is provided as an aqueous solution comprising the nitrate compound in a concentration of at least 1000 mg/L.

According to another preferred embodiment of the present disclosure, the nitrate compounds is provided as an aqueous solution comprising of from about 80 mg/L of nitrate to about 600 mg/L of nitrate. According to another preferred embodiment of the present disclosure, the nitrate compounds is provided as an aqueous solution comprising of from about 80 mg/L of nitrate to about 400 mg/L of nitrate. According to another preferred embodiment of the present disclosure, the nitrate compounds is provided as an aqueous solution comprising of from about 80 mg/L of nitrate to about 200 mg/L of nitrate. According to another preferred embodiment of the present disclosure, the nitrate compounds is provided as an aqueous solution comprising of from about 80 mg/L of nitrate to about 120 mg/L of nitrate. It is understood that said aqueous solution comprising nitrate may be prepared by dissolving an appropriate amount of nitrate in water or may occur naturally, e.g. as groundwater contaminated with nitrate.

It is understood that, if the nitrate is provided as an aqueous solution only, the concentration of the nitrate compound may be high: According to another preferred embodiment of the present disclosure, the nitrate compounds is provided as an aqueous solution comprising of from about 200 mg/L of nitrate to about 600 mg/L of nitrate. According to another preferred embodiment of the present disclosure, the nitrate compounds is provided as an aqueous solution comprising of from about 250 mg/L of nitrate to about 500 mg/L of nitrate. According to another preferred embodiment of the present disclosure, the nitrate compounds is provided as an aqueous solution comprising of from about 300 mg/L of nitrate to about 500 mg/L of nitrate. According to another preferred embodiment of the present disclosure, the nitrate compounds is provided as an aqueous solution comprising of from about 350 mg/L of nitrate to about 450 mg/L of nitrate.

On the other hand, it is understood that, if the nitrate is provided as an aqueous solution and via animal feed, the concentration of the nitrate compound may be lower: According to another preferred embodiment of the present disclosure, the nitrate compounds is provided as an aqueous solution comprising of from about 50 mg/L of nitrate to about 200 mg/L of nitrate. According to another preferred embodiment of the present disclosure, the nitrate compounds is provided as an aqueous solution comprising of from about 80 mg/L of nitrate to about 150 mg/L of nitrate. According to another preferred embodiment of the present disclosure, the nitrate compounds is provided as an aqueous solution comprising of from about 80 mg/L of nitrate to about 120 mg/L of nitrate.

According to a further preferred embodiment of the present disclosure, the nitrate compound is administered by both food and drink.

According to another preferred embodiment of the present disclosure, the nitrate compound is administered at least once per day, such as at least twice per day, or at least three times per day, or at least four times per day.

According to another preferred embodiment of the present disclosure, the subject has free access to feed and/or free access to drink. In other words, the nitrate compound is administered in a plurality of doses, i.e. whenever the subject eats or drinks. According to another preferred embodiment of the present disclosure, the nitrate compound is administered with every intake of feed and/or drink.

It is understood that fattening animals, such as pigs, may be fed 3 to 4 times per day. If the nitrate compound is administered via the feed or food, the nitrate compound is administered and ingested accordingly, i.e. in 3 or 4 doses per day.

The inventor of the present disclosure has surprisingly found that the nitrate compound may be administered conveniently with the feed or food. It is not necessary to monitor or control the ingestion of the nitrate compound because the subject automatically ingests the compound when feeding.

### Dosage of nitrate compound

According to a further preferred embodiment of the present disclosure, the nitrogen oxide compound for use in a method of treating and/or preventing a dysbacteriosis in a subject is a nitrate compound, in particular a nitrate compound as disclosed herein. The nitrate compound may be administered in the following doses:

According to another preferred embodiment of the present disclosure, the nitrate compound is administered in an amount of about 0.5 g/day or more based on the amount of nitrate.

According to another preferred embodiment of the present disclosure, the nitrate compound is administered in an amount of about 0.7 g/day or more based on the amount of nitrate. According to another preferred embodiment of the present disclosure, the nitrate compound is administered in an amount of about 1.0 g/day or more based on the amount of nitrate. According to another preferred embodiment of the present disclosure, the nitrate compound is administered in an amount of about 1.5 g/day or more based on the amount of nitrate, According to a further preferred embodiment of the present disclosure, the nitrate compound is administered in an amount of about 2.0 g/day or more based on the amount of nitrate. According to a yet further preferred embodiment of the present disclosure, the nitrate compound is administered in an amount of about 2.5 g/day or more.

The inventor of the present disclosure has found that an underdosing leads to an increase in bacterial infections, including bacterial infections with salmonella, *Brachyspira hyodysenteriae, Escherichia Coli, Lawsonia intracellularis* (Ileitis), in particular clostridial infections.

It is understood that, if the nitrate compound is administered once daily, a higher dose, such as 1. 5/day or more, 2.0 g/day or more, or 2.5 g/day or more, each based on the amount of nitrate, is needed. If, on the other hand, the nitrate compound is administered three or four times per day, the lower doses are be preferable.

According to another preferred embodiment of the present disclosure, the nitrate compound is administered in an amount of about 10 g/day or less based on the amount of nitrate. According to another preferred embodiment of the present disclosure, the nitrate compound is administered in an amount of about 8.0 g/day or less based on the amount of nitrate. According to another preferred embodiment of the present disclosure, the nitrate compound is administered in an amount of about 5.0 g/day or less based on the amount of nitrate. According to a further preferred embodiment of the present disclosure, the nitrate compound is administered in an amount of about 4.0 g/day or less based on the amount of nitrate. According to a yet further preferred embodiment of the present disclosure, the nitrate compound is administered in an amount of about 3.0 g/day or less based on the amount of nitrate.

In the event of an overdose, streptococcal infections occur more frequently, which are usually clinically visible in the form of meningitis (meningoencephalitis).

According to another preferred embodiment of the present disclosure, the nitrate compound is administered in an amount in the range of from about 0.5 g/day to about 10 g/day based on the amount of nitrate. According to another preferred embodiment of the present disclosure, the nitrate compound is administered in an amount in the range of from about 1.0 g/day to about 8.0 g/day based on the amount of nitrate. According to another preferred embodiment of the present disclosure, the nitrate compound is administered in an amount in the range of from about 1.0 g/day to about 5.0 g/day based on the amount of nitrate. According to another preferred embodiment of the present disclosure, the nitrate compound is administered in an amount in the range of from about 1.5 g/day to about 4.0 g/day based on the amount of nitrate. According to a further preferred embodiment of the present disclosure, the nitrate compound is administered in an amount in the range of from about 2.0 g/day to about 4.0 g/day based on the amount of nitrate. According to a yet further preferred embodiment of the present disclosure, the nitrate compound is administered preferably in an amount in the range of from about 2.5 g/day to about 3.0 g/day based on the amount of nitrate.

The inventor of the present disclosure surprisingly found that the above doses are highly efficient in treating and/or preventing dysbacteriosis in a subject without adverse events.

The dose of nitrate compound may also be given in relation to the body weight of the subject:

According to another preferred embodiment of the present disclosure, the nitrate compound is administered in an amount of about 10 mg/kg/day or more based on the amount of nitrate. According to a further preferred embodiment of the present disclosure, the nitrate compound is administered in an amount of about 15 mg/kg/day or more based on the amount of nitrate. According to a yet further preferred embodiment of the present disclosure, the nitrate compound is administered in an amount of about 17 mg/kg/day or more based on the amount of nitrate. According to another preferred embodiment of the present disclosure, the nitrate compound is administered in an amount of about 20 mg/kg/day or more based on the amount of nitrate.

According to another preferred embodiment of the present disclosure, the nitrate compound is administered in an amount of about 100 mg/kg/day or less based on the amount of nitrate. According to another preferred embodiment of the present disclosure, the nitrate compound is administered in an amount of about 75 mg/kg/day or more based on the amount of nitrate. According to a further preferred embodiment of the present disclosure, the nitrate compound is administered in an amount of about 60 mg/kg/day or less based on the amount of nitrate. According to a yet further preferred embodiment of the present disclosure, the nitrate compound is administered in an amount of about 50 mg/kg/day or less based on the amount of nitrate.

According to another preferred embodiment of the present disclosure, the nitrate compound is administered in an amount in the range of from about 10 mg/kg/day to about 100 mg/kg/day based on the amount of nitrate. According to another preferred embodiment of the present disclosure, the nitrate compound is administered in an amount in the range of from about 15 mg/kg/day to about 75 mg/kg/day based on the amount of nitrate. According to a further preferred embodiment of the present disclosure, the nitrate compound is administered in an amount in the range of from about 17 mg/kg/day to about 60 mg/kg/day based on the amount of nitrate. According to a yet further preferred embodiment of the present disclosure, the nitrate compound is administered in an amount in the range of from about 20 mg/kg/day to about 50 mg/kg/day based on the amount of nitrate.

### Dysbacteriosis and symptoms thereof

According to another preferred embodiment of the present disclosure, the dysbacteriosis is caused by a bacterium from the family of *Clostridiaceae,* or the family of *Enterobacteriaceae,* or the family of *Desulfovibrionaceae,* or the family of *Brachyspiraceae.*

A particular preferred bacterium from the family of *Clostridiaceae* is of the genus *Clostridium,* more preferably a from the species of *Clostridium perfringens,* or *Clostridium botulinum.*

A particular preferred bacterium from the family of *Enterobacteriaceae* is of the genus *Salmonella* or of the genus *Escherichia,* more preferably a from the species of *Escherichia coli* or *Salmonella bongori,* or *Salmonella enterica,* or *Salmonella choleraesuis,* or *Salmonella thyphisuis.*

A particular preferred bacterium from the family of *Desulfovibrionaceae* is of the genus *Lawsonia,* more preferably from the species of *Lawsonia intracellularis.*

According to another preferred embodiment of the present disclosure, the dysbacteriosis is caused by a bacterium from the genus of *Clostridium* or from the genus of *Escherichia* or from the genus of *Salmonella,* or from the genus of *Lawsonia.*

According to another preferred embodiment of the present disclosure, the dysbacteriosis is caused by a bacterium from the species of *Clostridium perfringens, Clostridium botulinum, Escherichia coli,* or *Lawsonia intracellularis.*

According to another preferred embodiment of the present disclosure, the a preferred bacterium from the family of *Brachyspiraceae* is of the genus *Brachyspira.* According to another preferred embodiment of the present disclosure, the dysbacteriosis is caused by a bacterium from the species of *Brachyspira hyodysenteriae.*

According to another preferred embodiment of the present disclosure, the dysbacteriosis is caused by a bacterium from the genus of *Clostridium* or from the family of *Escherichia.*

According to another preferred embodiment of the present disclosure, the dysbacteriosis is caused by a bacterium from the species of *Clostridium perfringens, Clostridium botulinum,* or *Escherichia coli.*

The inventor of the present disclosure has surprisingly found that a dysbacteriosis associated with the above pathogenic bacteria could be substantially reduced (see example 1). Therefore, not only the number of deaths associated with bacterial infections, but also the need for antibiotics and costs for veterinary professionals can be substantially reduced. Also the feed efficiency, that is the amount of feed needed for gaining 1 kg of body weight of the subject, is significantly improved.

### Post-weaning diarrhea

According to another preferred embodiment of the present disclosure, the dysbacteriosis is associated with acute diarrhea, preferably wherein the dysbacteriosis is associated with acute post-weaning diarrhea.

The inventor of the present disclosure surprisingly found that dysbacteriosis-related illnesses, such as diarrhea, can be efficiently prevented and/or treated thus reducing the number of piglet deaths and veterinary costs.

According to another preferred embodiment of the present disclosure, treating and/or preventing acute post-weaning diarrhea may require adjusting the dose of the nitrate compound as disclosed herein as follows.

According to another preferred embodiment of the present disclosure, the nitrate compound is administered in an amount of about 50 mg/kg/day or more based on the amount of nitrate. According to another preferred embodiment of the present disclosure, the nitrate compound is administered in an amount of about 60 mg/kg/day or more based on the amount of nitrate. According to another preferred embodiment of the present disclosure, the nitrate compound is administered in an amount of about 80 mg/kg/day or more based on the amount of nitrate. According to another preferred embodiment of the present disclosure, the nitrate compound is administered even more preferably in an amount of about 100 mg/kg/day or more based on the amount of nitrate. According to a yet further preferred embodiment of the present disclosure, the nitrate compound is administered in an amount of about 120 mg/kg/day or more based on the amount of nitrate.

According to another preferred embodiment of the present disclosure, the nitrate compound is administered the nitrate compound is administered in an amount of about 350 mg/kg/day or less based on the amount of nitrate. According to another preferred embodiment of the present disclosure, the nitrate compound is administered in an amount of about 325 mg/kg/day or less based on the amount of nitrate. According to another preferred embodiment of the present disclosure, the nitrate compound is administered in an amount of about 300 mg/kg/day or less based on the amount of nitrate. According to a further preferred embodiment of the present disclosure, the nitrate compound is administered in an amount of about 275 mg/kg/day or less based on the amount of nitrate. According to a yet further preferred embodiment of the present disclosure, the nitrate compound is administered in an amount of about 200 mg/kg/day or less based on the amount of nitrate.

According to another preferred embodiment of the present disclosure, the nitrate compound is administered in an amount in the range of from about 50 mg/kg/day to about 350 mg/kg/day based on the amount of nitrate. According to another preferred embodiment of the present disclosure, the nitrate compound is administered in an amount in the range of from about 60 mg/kg/day to about 325 mg/kg/day based on the amount of nitrate. According to another preferred embodiment of the present disclosure, the nitrate compound is administered in an amount in the range of from about 80 mg/kg/day to about 300 mg/kg/day based on the amount of nitrate. According to a further preferred embodiment of the present disclosure, the nitrate compound is administered in an amount in the range of from about 100 mg/kg/day to about 275 mg/kg/day based on the amount of nitrate. According to a yet further preferred embodiment of the present disclosure, the nitrate compound is administered in an amount in the range of from about 120 mg/kg/day to about 250 mg/kg/day or less based on the amount of nitrate.

The inventor of the present disclosure has surprisingly found that the above doses are highly effective in treating post-weaning diarrhea thus supporting the welfare of the subjects, such as piglets, preventing gut damage, reducing animal death, and ensuring sustainable animal growth.

According to another preferred embodiment of the present disclosure, the nitrate compound is administered at least once per day. According to another preferred embodiment of the present disclosure, the nitrate compound is administered of from 2 to 6 times, preferably three or four times.

The inventor of the present disclosure has surprisingly found that post-weaning diarrhea can be efficiently treated by administering only up to 6 doses of the nitrate compound. Even after stopping the treatment, no further post-weaning diarrhea was observed.

According to another preferred embodiment of the present disclosure, the nitrate compound is administered at least once daily, preferably once, twice, or three times per day, more preferably twice daily.

### Definitions

As used herein, the term "pharmaceutically acceptable cation" refers a cation that is not toxic to mammals. Preferred pharmaceutically acceptable cations are lithium, sodium, potassium, aluminium, and zinc, cations made from organic bases such as choline, diethanolamine, morpholine, ammonium salts, quaternary salts such as tetramethylammonium salt; amino acid addition salts such as salts with glycine and arginine.

As used herein, the concentration of nitrate in an aqueous solution is determined according to DIN ISO 15923-1 (D 49) of July 20214.

As used herein, the dose of the nitrate compound is indicated based on the amount of nitrate, i.e. based on the amount of the nitrate ion. In other words, it is understood that the mass of the pharmaceutically acceptable cation is not taken into account when determining the dose of nitrate compound. For example, if the nitrate compound is potassium nitrate, administering an amount of 1 g of nitrate compound based on the amount of nitrate requires administering 1.63 g of potassium nitrate.

In other word, 1.63 g of potassium nitrate correspond to 1.0 g of nitrate compound based on the amount of nitrate. 1.37 g of sodium nitrate correspond to 1.0 g of nitrate compound based on the amount of nitrate.

The term "based on the amount of nitrate" corresponds to the amount of nitrate ion contained in the nitrate compound.

If an amount is given in the unit "mg/kg/day", it is understood that the daily dose of nitrate compound based on the amount of nitrate is given in relation to the body weight of the subject. The dose is calculated as $\frac{amount of nitrate / day}{body weight of the subject}$

As used herein, the term "fattening animal" refers to an animal in any stage of a fattening process. Accordingly, a fattening animal may be a piglet as disclosed herein, such as a weaning piglet, a pig in the nursery stage as disclosed herein, a pig in the grower stage as disclosed herein, or a pig in the finisher stage as disclosed herein.

As used herein, the term "breeding animal" refers to an animal that is not part of a fattening process, but is used for breeding. According to a preferred embodiment, the breeding animal is a breeding sow.

Herein, the terms "amount" and "dose" of nitrogen oxide compound are used interchangeably.

The terms "a", "an", and "the" refer to one or more.

Where the term "comprising" is used in the present description and claims, it does not exclude other elements. For the purposes of the present disclosure, the term "consisting of" is considered to be a preferred embodiment of the term "comprising". If a group is defined to comprise at least a certain number of embodiments herein, this is also to be understood to disclose a group, which preferably consists only of these embodiments. Furthermore, if a composition is defined using the term "comprising", it may additionally comprise other elements not explicitly listed, however, not further amounts of an element listed.

The term "about" in conjunction with a numerical value refers to normal deviations of said numerical value. It is to be understood that the term "about" can mean a deviation of ± 10 %, preferably ± 5 %, more preferably ± 2.5 % of said numeric value as indicated.

### Examples

### Example 1 - Treatment and prevention of dysbacteriosis in pigs

Potassium nitrate (Nova N-K; obtained from ICL Growing Solutions, part of Everris International B.V, Netherlands) was mixed with animal feed, either manually or automatically via an additive feeder integrated into the feeding system.

Pigs received three or four meals per day and water (containing about 100 mg/L of nitrate determined according to DIN ISO 15923-1 (D 49) of July 20214) ad libitum. It is understood that a pig drinks about 1 L of water per 10 kg of body weight per day (a smaller pig weighing 25 kg drinks about 2.5 L of water per day; a bigger pig weighing 100 kg drinks about 10 L of water per day).

Pigs received about 1 g of potassium nitrate per 20-30 kg of body weight per day with the feed, i.e. 20-31 mg/kg/day based on the amount of nitrate.

In total (feed and drink together), each pig received about the following amounts of nitrate

**Table 1. Dosing of nitrate**

| | small pigs (about 25 kg body weight) | big pigs (about 100 kg body weight) |
|---|---|---|
| dose of nitrate over feed | 1.12 g of KNO₃ per day (= 0.69 g of NO₃⁻ per day) | 2.95 g of KNO₃ per day (= 1.81 g of NO₃⁻ per day) |
| dose of nitrate over drink | 0.25 g of NO₃⁻ per day | 1 g of NO₃⁻ per day |
| total | 0.94 g of NO₃⁻ per day | 2.81 g of NO₃⁻ per day |

The following effects were observed:

### Salmonella

Samples are routinely taken at the slaughterhouse as part of a monitoring program and tested for salmonella (ELISA). Positive samples are greatly reduced for the above animals that received nitrate over animals that were not fed with nitrate.

### Lawsonia intracellularis (Ileitis)

The pathogen is present in almost all pig farms. *Lawsonia intracellularis* causes proliferative inflammation of the ileum mucosa (ileitis).

Typically, an infection with *Lawsonia intracellularis* manifests clinically in the form of proliferative hemorrhagic enteropathy (PHE). For this reason, all pigs are typically vaccinated to prevent an infection with *Lawsonia intracellularis.*

However, the pigs that received nitrate were not vaccinated. Nevertheless, no infection was observed. Thus, the cost of medicines is significantly reduced fallen by about 80-90% due to feeding nitrate.

### Restlessness/ cannibalism

In big herds, such as in intense livestock farming facilities, pigs are very restless and unhappy. As a result, cannibalism (biting each other's tails) is particularly noticeable.

However, the above pigs fed with nitrate are very calm and relaxed. Cannibalism is very rarely observed.

Therefore, the common practice of tail-docking, which causes acute trauma and pain, to prevent cannibalism is not necessary. Therefore, the present disclosure also complies with the EU and German policy planning to ban this procedure.

Without wishing to be bound by theory, it appears that metabolic problems in pig can be prevented/remedied by feeding nitrate. This may improve the general well-being of the pigs, thus reducing aggression, and effectively combats the causes of SINS.

### Meat content

The ratio of lean meat to fat is an important parameter for the payment of the pigs. This is why these parameters are determined automatically at the slaughterhouse. High lean meat percentages are rewarded with price premiums, while deductions are made for fatter pigs.

The above nitrate-fed pigs have very high lean meat values with low fat content compared to pigs which have not received nitrate.

### Feed conversion

Feed conversion describes how many kilograms of feed are needed to produce one kilogram of meat. Considered over the entire production, feed is the largest cost factor at 70-80%, so feed conversion is a decisive factor for profitability but also for the CO₂ footprint.

Pigs which have not received nitrate during the fattening phase had a feed conversion of about 2.74.

The above pigs which have received nitrate during the fattening phase had a feed conversion of about 2.66.

Thus, administration of nitrate improved feed conversion.

### Example 2 - Treatment of post-weaning diarrhea in piglets

14 piglets (body weight about 7 kg) were separated from their mothers and the diet was changed to solid feed (prestarter).

About 7.5 g of potassium nitrate (Nova N-K; obtained from ICL Growing Solutions, part of Everris International B.V, Netherlands) was dissolved in 1 L of a commercially available Cola drink to prepare a potassium nitrate solution.

Upon observing post-weaning diarrhea, the piglets each received about 70 mL of the potassium nitrate solution twice per day.

After 3 to 4 doses of the potassium nitrate solution, i.e. after 1.5 to 2 days, the post-weaning diarrhea stopped and the piglets resumed normal growth. Thus, the duration of post-weaning diarrhea was considerably reduced.

The piglets did not show signs of adverse events.

### Example 3 - Prevention of post-weaning diarrhea

A group of sows (body weight about 150 kg to about 250 kg) was divided into an experimental group and a control group one week before giving birth.

The sows in the experimental group received about 15 g of potassium nitrate (Nova N-K; obtained from ICL Growing Solutions, part of Everris International B.V, Netherlands) per day once daily. No further nitrate was given after giving birth. The sows in the control group did not receive nitrate.

After weaning, no post-weaning diarrhea was observed in the piglets from the sows in the experimental group which had received potassium nitrate before farrowing. On the other hand, the piglets of the sows that had not received nitrate (control group) showed typical post-weaning diarrhea.

### EMBODIMENTS

1. A nitrogen oxide compound for use in a method of treating and/or preventing a dysbacteriosis in a subject.
2. The nitrogen oxide compound for use in a method of treating and/or preventing a dysbacteriosis according to embodiment 1, wherein the nitrogen oxide compound is a nitrate compound and/or a nitrite compound.
3. The nitrogen oxide compound for use in a method of treating and/or preventing a dysbacteriosis according to any one of embodiments 1 or 2, wherein the nitrogen oxide compound is a nitrate compound, preferably an alkali nitrate or earth alkali nitrate.
4. The nitrogen oxide compound for use in a method of treating and/or preventing a dysbacteriosis according to any one of embodiments 1 to 3, wherein the nitrate compound is selected from the group consisting of ammonium nitrate (NH₄NO₃), potassium nitrate (KNO₃), sodium nitrate (NaNO₃), calcium nitrate (Ca(NO₃)₂), magnesium nitrate (Mg(NO₃)₂), zinc nitrate (Zn(NO₃)₂), copper nitrate (Cu(NO₃)₂), silver nitrate (AgNO₃), and a mixture thereof.
5. The nitrogen oxide compound for use in a method of treating and/or preventing a dysbacteriosis according to any one of embodiments 1 to 4, wherein the nitrate compound is potassium nitrate (KNO₃), or sodium nitrate (NaNO₃), or a mixture thereof.
6. The nitrogen oxide compound for use in a method of treating and/or preventing a dysbacteriosis according to any one of embodiments 1 or 2, wherein the nitrogen oxide compound is a nitrite compound, preferably an alkali nitrite or earth alkali nitrite.
7. The nitrogen oxide compound for use in a method of treating and/or preventing a dysbacteriosis according to any one of embodiments 1, 2, or 6, wherein the nitrite compound is selected from the group consisting of ammonium nitrite (NH₄NO₂), potassium nitrite (KNO₃), sodium nitrite (NaNO₂), calcium nitrite (Ca(NO₂)₂), magnesium nitrite (Mg(NO₂)₂), zinc nitrite (Zn(NO₂)₂), copper nitrite (Cu(NO₂)₂), silver nitrite (AgNO₂), and a mixture thereof.
8. The nitrogen oxide compound for use in a method of treating and/or preventing a dysbacteriosis according to any one of embodiments 1, 2, 6, or 7, wherein the nitrite compound is potassium nitrite (KNO₂), or sodium nitrite (NaNO₂), or a mixture thereof.
9. The nitrogen oxide compound for use in a method of treating and/or preventing a dysbacteriosis according to any one of claim 1 to 8, wherein the subject is an animal, preferably wherein the animal is not a ruminant, more preferably wherein the subject is a pig or a poultry, most preferably a pig.
10. The nitrogen oxide compound for use in a method of treating and/or preventing a dysbacteriosis according to any one of embodiments 1 to 9, wherein the nitrogen oxide compound is administered orally.
11. The nitrogen oxide compound for use in a method of treating and/or preventing a dysbacteriosis according to any one of embodiments 1 to 7, wherein the dysbacteriosis is caused by a bacterium from the family of *Brachyspiraceae, Clostridiaceae* or the family of *Enterobacteriaceae* or the family of *Desulfovibrionaceae.*
12. The nitrogen oxide compound for use in a method of treating and/or preventing a dysbacteriosis according to any one of embodiments 1 to 8, wherein the dysbacteriosis is caused by a bacterium from the genus of *Brachyspira,* or from the genus of *Clostridium* or from the genus of *Escherichia* or from the genus of *Salmonella,* or from the genus of *Lawsonia.*
13. The nitrogen oxide compound for use in a method of treating and/or preventing a dysbacteriosis according to any one of embodiments 1 to 12, wherein the dysbacteriosis is caused by a bacterium from the species of *Clostridium perfringens, Clostridium botulinum, Escherichia coli,* or *Lawsonia intracellularis,* or *Brachyspira hyodysenteriae.*
14. The nitrogen oxide compound for use in a method of treating and/or preventing a dysbacteriosis according to any one of embodiments 2 to 5 or 9 to 13, wherein the nitrate compound is administered in an amount of about 0.5 g/day or more based on the amount of nitrate.
15. The nitrogen oxide compound for use in a method of treating and/or preventing a dysbacteriosis according to any one of embodiments 2 to 5 or 9 to 14, wherein the nitrate compound is administered in an amount of about 0.7 g/day or more based on the amount of nitrate, preferably in an amount of about 1.0 g/day or more based on the amount of nitrate, more preferably in an amount of about 1.5 g/day or more based on the amount of nitrate, yet more preferably in an amount of about 2.0 g/day or more based on the amount of nitrate, most preferably in an amount of about 2.5 g/day or more.
16. The nitrogen oxide compound for use in a method of treating and/or preventing a dysbacteriosis according to any one of embodiments 2 to 5 or 9 to 15, wherein the nitrate compound is administered in an amount of about 10 g/day or less based on the amount of nitrate, preferably in an amount of about 8.0 g/day or less based on the amount of nitrate, more preferably in an amount of about 5.0 g/day or less based on the amount of nitrate, yet more preferably in an amount of about 4.0 g/day or less based on the amount of nitrate, most preferably in an amount of about 3.0 g/day or less based on the amount of nitrate.
17. The nitrogen oxide compound for use in a method of treating and/or preventing a dysbacteriosis according to any one of embodiments 2 to 5 or 9 to16, wherein the nitrate compound is administered in an amount in the range of from about 0.5 g/day to about 10 g/day based on the amount of nitrate, preferably in an amount in the range of from about 1.0 g/day to about 8.0 g/day based on the amount of nitrate, more preferably in an amount in the range of from about 1.0 g/day to about 5.0 g/day based on the amount of nitrate, even more preferably in an amount in the range of from about 1.5 g/day to about 4.0 g/day based on the amount of nitrate, yet more preferably in an amount in the range of from about 2.0 g/day to about 4.0 g/day based on the amount of nitrate, most preferably in an amount in the range of from about 2.5 g/day to about 3.0 g/day based on the amount of nitrate.
18. The nitrogen oxide compound for use in a method of treating and/or preventing a dysbacteriosis according to any one of embodiments 2 to 5 or 9 to 17, wherein the nitrate compound is administered in an amount of about 10 mg/kg/day or more, preferably in an amount of about 15 mg/kg/day or more, more preferably in an amount of about 17 mg/kg/day or more, most preferably in an amount of about 20 mg/kg/day or more, each based on the amount of nitrate.
19. The nitrogen oxide compound for use in a method of treating and/or preventing a dysbacteriosis according to any one of embodiments 2 to 5 or 9 to 18, wherein the nitrate compound is administered in an amount of about 100 mg/kg/day or less, preferably in an amount of about 75 mg/kg/day or more, more preferably in an amount of about 60 mg/kg/day or less, most preferably in an amount of about 50 mg/kg/day or less, each based on the amount of nitrate.
20. The nitrogen oxide compound for use in a method of treating and/or preventing a dysbacteriosis according to any one of embodiments 2 to 5 or 9 to 119, wherein the nitrate compound is administered in an amount in the range of from about 10 mg/kg/day to about 100 mg/kg/day, preferably in an amount in the range of from about 15 mg/kg/day to about 75 mg/kg/day, more preferably in an amount in the range of from about 17 mg/kg/day to about 60 mg/kg/day, most preferably in an amount in the range of from about 20 mg/kg/day to about 50 mg/kg/day, each based on the amount of nitrate.
21. The nitrogen oxide compound for use in a method of treating and/or preventing a dysbacteriosis according to any one of embodiments 1 to 20, wherein the nitrogen oxide compound is administered at least once per day, such as at least twice per day, or at least three times per day, or at least four times per day.
22. The nitrogen oxide compound for use in a method of treating and/or preventing a dysbacteriosis according to any one of embodiments 1 to 21, wherein the nitrogen oxide compound is administered with a meal and/or with drink.
23. The nitrogen oxide compound for use in a method of treating and/or preventing a dysbacteriosis according to any one of embodiments 1 to 22, wherein the dysbacteriosis is associated with acute diarrhea, preferably wherein the dysbacteriosis is associated with acute post-weaning diarrhea.
24. The nitrogen oxide compound for use in a method of treating and/or preventing dysbacteriosis according to claim 23, wherein the nitrogen oxide compound is a nitrate compound and is administered in an amount of about 50 mg/kg/day or more, preferably in an amount of about 60 mg/kg/day or more, more preferably in an amount of about 70 mg/kg/day or more, even more preferably in an amount of about 80 mg/kg/day or more, most preferably in an amount of about 85 mg/kg/day or more, each based on the amount of nitrate.
25. The nitrogen oxide compound for use in a method of treating and/or preventing dysbacteriosis according to claim 23 or 24, wherein the nitrogen oxide compound is a nitrate compound and is administered in an amount of about 250 mg/kg/day or less, preferably in an amount of about 225 mg/kg/day or less, more preferably in an amount of about 200 mg/kg/day or less, even more preferably in an amount of about 175 mg/kg/day or less, most preferably in an amount of about 150 mg/kg/day or less, each based on the amount of nitrate.
26. The nitrogen oxide compound for use in a method of treating and/or preventing dysbacteriosis according to any one of embodiments 23 to 25, wherein the nitrogen oxide compound is a nitrate compound and is administered in an amount in the range of from about 50 mg/kg/day to about 250 mg/kg/day, preferably in an amount in the range of from about 60 mg/kg/day to about 225 mg/kg/day, more preferably in an amount in the range of from about 70 mg/kg/day to about 200 mg/kg/day, even more preferably in an amount in the range of from about 80 mg/kg/day to about 175 mg/kg/day, most preferably in an amount in the range of from about 85 mg/kg/day to about 150 mg/kg/day or less, each based on the amount of nitrate.
27. The nitrogen oxide compound for use in a method of treating and/or preventing dysbacteriosis according to any one of embodiments 23 to 26, wherein the nitrogen oxide compound is a nitrate compound and is administered of from 2 to 6 times, preferably three or four times.
28. The nitrogen oxide compound for use in a method of treating and/or preventing dysbacteriosis according to any one of embodiments 23 to 27, wherein the nitrogen oxide compound is a nitrate compound and administered at least once daily, preferably once, twice, or three times per day, more preferably twice daily.

## Claims

1. A nitrogen oxide compound for use in a method of treating and/or preventing a dysbacteriosis in a subject.

2. The nitrogen oxide compound for use in a method of treating and/or preventing a dysbacteriosis according to claim 1, wherein the nitrogen oxide compound is a nitrate compound and/or a nitrite compound.

3. The nitrogen oxide compound for use in a method of treating and/or preventing a dysbacteriosis according to any one of claims 1 or 2, wherein the nitrogen oxide compound is a nitrate compound, preferably an alkali nitrate or earth alkali nitrate, more preferably the nitrate compound is selected from the group consisting of ammonium nitrate (NH₄NO₃), potassium nitrate (KNO₃), sodium nitrate (NaNO₃), calcium nitrate (Ca(NO₃)₂), magnesium nitrate (Mg(NO₃)₂), zinc nitrate (Zn(NO₃)₂), copper nitrate (Cu(NO₃)₂), silver nitrate (AgNO₃), and a mixture thereof, most preferably wherein the nitrate compound is potassium nitrate (KNO₃), or sodium nitrate (NaNO₃), or a mixture thereof.

4. The nitrogen oxide compound for use in a method of treating and/or preventing a dysbacteriosis according to any one of claims 1 or 2, wherein the nitrogen oxide compound is a nitrite compound, preferably an alkali nitrite or earth alkali nitrite, more preferably wherein the nitrite compound is selected from the group consisting of ammonium nitrite (NH₄NO₂), potassium nitrite (KNO₃), sodium nitrite (NaNO₂), calcium nitrite (Ca(NO₂)₂), magnesium nitrite (Mg(NO₂)₂), zinc nitrite (Zn(NO₂)₂), copper nitrite (Cu(NO₂)₂), silver nitrite (AgNO₂), and a mixture thereof, most preferably wherein the nitrite compound is potassium nitrite (KNO₂), or sodium nitrite (NaNO₂), or a mixture thereof.

5. The nitrogen oxide compound for use in a method of treating and/or preventing a dysbacteriosis according to any one of claim 1 to 4, wherein the subject is an animal, preferably wherein the animal is not a ruminant, more preferably wherein the subject is a pig or a poultry, most preferably a pig.

6. The nitrogen oxide compound for use in a method of treating and/or preventing a dysbacteriosis according to any one of claims 1 to 5, wherein the nitrogen oxide compound is administered orally.

7. The nitrogen oxide compound for use in a method of treating and/or preventing a dysbacteriosis according to any one of claims 1 to 6, wherein the dysbacteriosis is caused by a bacterium from the family of *Brachyspiraceae, Clostridiaceae* or the family of *Enterobacteriaceae* or the family of *Desulfovibrionaceae,* preferably wherein the dysbacteriosis is caused by a bacterium from the genus of *Brachyspira,* or from the genus of *Clostridium* or from the genus of *Escherichia* or from the genus of *Salmonella,* or from the genus of *Lawsonia,* most preferably wherein the dysbacteriosis is caused by a bacterium from the species of *Clostridium perfringens, Clostridium botulinum, Escherichia coli,* or *Lawsonia intracellularis,* or *Brachyspira hyodysenteriae.*

8. The nitrogen oxide compound for use in a method of treating and/or preventing a dysbacteriosis according to any one of claims 1 to 3 or 5 to 7, wherein
a. the nitrate compound is administered in an amount of about 0.5 g/day or more based on the amount of nitrate, preferably in an amount of about 0.7 g/day or more based on the amount of nitrate, more preferably in an amount of about 1.0 g/day or more based on the amount of nitrate, more preferably in an amount of about 1.5 g/day or more based on the amount of nitrate, yet more preferably in an amount of about 2.0 g/day or more based on the amount of nitrate, most preferably in an amount of about 2.5 g/day or more; and/or
b. the nitrate compound is administered in an amount of about 10 g/day or less based on the amount of nitrate, preferably in an amount of about 8.0 g/day or less based on the amount of nitrate, more preferably in an amount of about 5.0 g/day or less based on the amount of nitrate, yet more preferably in an amount of about 4.0 g/day or less based on the amount of nitrate, most preferably in an amount of about 3.0 g/day or less based on the amount of nitrate; and/or
c. the nitrate compound is administered in an amount in the range of from about 0.5 g/day to about 10 g/day based on the amount of nitrate, preferably in an amount in the range of from about 1.0 g/day to about 8.0 g/day based on the amount of nitrate, more preferably in an amount in the range of from about 1.0 g/day to about 5.0 g/day based on the amount of nitrate, even more preferably in an amount in the range of from about 1.5 g/day to about 4.0 g/day based on the amount of nitrate, yet more preferably in an amount in the range of from about 2.0 g/day to about 4.0 g/day based on the amount of nitrate, most preferably in an amount in the range of from about 2.5 g/day to about 3.0 g/day based on the amount of nitrate.

9. The nitrogen oxide compound for use in a method of treating and/or preventing a dysbacteriosis according to any one of claims 1 to 3 or 5 to 8, wherein
a. the nitrate compound is administered in an amount of about 10 mg/kg/day or more, preferably in an amount of about 15 mg/kg/day or more, more preferably in an amount of about 17 mg/kg/day or more, most preferably in an amount of about 20 mg/kg/day or more, each based on the amount of nitrate; and or
b. the nitrate compound is administered in an amount of about 100 mg/kg/day or less, preferably in an amount of about 75 mg/kg/day or more, more preferably in an amount of about 60 mg/kg/day or less, most preferably in an amount of about 50 mg/kg/day or less, each based on the amount of nitrate; and/or
c. the nitrate compound is administered in an amount in the range of from about 10 mg/kg/day to about 100 mg/kg/day, preferably in an amount in the range of from about 15 mg/kg/day to about 75 mg/kg/day, more preferably in an amount in the range of from about 17 mg/kg/day to about 60 mg/kg/day, most preferably in an amount in the range of from about 20 mg/kg/day to about 50 mg/kg/day, each based on the amount of nitrate.

10. The nitrogen oxide compound for use in a method of treating and/or preventing a dysbacteriosis according to any one of claims 1 to 9, wherein the nitrogen oxide compound is administered at least once per day, such as at least twice per day, or at least three times per day, or at least four times per day

11. The nitrogen oxide compound for use in a method of treating and/or preventing a dysbacteriosis according to any one of claims 1 to 10, wherein the nitrogen oxide compound is administered with a meal and/or with drink.

12. The nitrogen oxide compound for use in a method of treating and/or preventing a dysbacteriosis according to any one of claims 1 to 11, wherein the dysbacteriosis is associated with acute diarrhea, preferably wherein the dysbacteriosis is associated with acute post-weaning diarrhea.

13. The nitrogen oxide compound for use in a method of treating and/or preventing dysbacteriosis according to claim 12, wherein the nitrogen oxide compound is a nitrate compound and is administered
a. in an amount of about 50 mg/kg/day or more, preferably in an amount of about 60 mg/kg/day or more, more preferably in an amount of about 70 mg/kg/day or more, even more preferably in an amount of about 80 mg/kg/day or more, most preferably in an amount of about 85 mg/kg/day or more, each based on the amount of nitrate; and/or
b. in an amount of about 250 mg/kg/day or less, preferably in an amount of about 225 mg/kg/day or less, more preferably in an amount of about 200 mg/kg/day or less, even more preferably in an amount of about 175 mg/kg/day or less, most preferably in an amount of about 150 mg/kg/day or less, each based on the amount of nitrate; and/or
c. in an amount in the range of from about 50 mg/kg/day to about 250 mg/kg/day, preferably in an amount in the range of from about 60 mg/kg/day to about 225 mg/kg/day, more preferably in an amount in the range of from about 70 mg/kg/day to about 200 mg/kg/day, even more preferably in an amount in the range of from about 80 mg/kg/day to about 175 mg/kg/day, most preferably in an amount in the range of from about 85 mg/kg/day to about 150 mg/kg/day or less, each based on the amount of nitrate.

14. The nitrogen oxide compound for use in a method of treating and/or preventing dysbacteriosis according to any one of claims 1 to 13, wherein the nitrogen oxide compound is a nitrate compound and is administered of from 2 to 6 times, preferably three or four times.

15. The nitrogen oxide compound for use in a method of treating and/or preventing dysbacteriosis according to any one of claims 1 to 13, wherein the nitrogen oxide compound is administered with every feed and/or drink.
